# EUROPEAN PATENT SPECIFICATION

(11) **EP 2 006 682 B1**
(45) Date of publication and mention of the grant of the patent: **04.06.2014**
(21) Application number: 07739308.0
(22) Date of filing: 22.03.2007
(51) Int. Cl.: G01N 33/566

(54) **DIAGNOSIS OF ACUTE ENTERITIS BY DETERMINATION OF INTESTINAL FATTY ACID-BINDING PROTEIN IN THE BLOOD**
DIAGNOSE AKUTER ENTERITIS MITTELS BESTIMMUNG DES INTESTINALEN FETTSÄUREBINDENDEN PROTEINS IM BLUT
DIAGNOSTIC DE L'ENTERITE AIGUE PAR LA DETERMINATION DE LA PROTEINE INTESTINALE DE LIAISON AUX ACIDES GRAS DANS LE SANG

(30) Priority: 22.03.2006 JP 2006079565
(43) Date of publication of application: 24.12.2008
(73) Proprietor: DS Pharma Biomedical Co., Ltd., Suita Osaka 564-0063 (JP)
(72) Inventor: KANDA, Tatsuo c/o NIIGATA UNIVERSITY, Chuo-ku Niigata-shi, Niigata 951-8510 (JP); FUJII, Hiroshi c/o SHINSHU UNIVERSITY, Kamiina Country Nagano 399-4598 (JP); FUNAOKA, Hiroyuki, Suita-shi, Osaka 564-0053 (JP); KAJIURA, Satoshi, Suita-shi, Osaka 564-0053 (JP); OHKARU, Yasuhiko, Suita-shi, Osaka 564-0053 (JP)
(74) Representative: Vossius & Partner
(86) International application number: PCT/JP2007/055865
(87) International publication number: WO 2007/119481

(56) References cited:
- WO-A1-93/08276
- WO-A1-03/083486
- MORARIU AURORA M ET AL: "Dexamethasone: Benefit and prejudice for patients undergoing on-pump coronary artery bypass grafting - A study on myocardial, pulmonary, renal, intestinal, and hepatic injury" CHEST, vol. 128, no. 4, October 2005 (2005-10), pages 2677-2687, XP002553747 ISSN: 0012-3692
- Cell Sciences: "Human Intenstinal fatty acid binding protein (I-FABP) ELISA kit" Catalog HK406: Hycult biotechnology, [Online] 2005, XP002553748 Retrieved from the Internet: URL:http://www.cellsciences.com/PDF/HK406. pdf> [retrieved on 2009-11-04]
- YEUNG CHUN-YAN ET AL: "Serum cytokines in differentiating between viral and bacterial enterocolitis" ANNALS OF TROPICAL PAEDIATRICS, vol. 24, no. 4, December 2004 (2004-12), pages 337-343, XP9125161 ISSN: 0272-4936
- PELSERS M M ET AL: "Intestinal-type and liver-type fatty acid-binding protein in the intestine. Tissue distribution and clinical utility, Oct. 2003, ACCESSION: NM 001443" CLINICAL BIOCHEMISTRY, ELSEVIER INC, US, CA, vol. 36, no. 7, 1 October 2003 (2003-10-01), pages 529-535, XP002986944 ISSN: 0009-9120
- BOS JOHN ET AL: "Fatal necrotizing colitis following a foodborne outbreak of enterotoxigenic Clostridium perfringens type A infection" CLINICAL INFECTIOUS DISEASES, vol. 40, no. 10, May 2005 (2005-05), pages E78-E83, XP002553783 ISSN: 1058-4838
- BLAKELOCK RUSSELL T ET AL: "Infection and the gut." SEMINARS IN PEDIATRIC SURGERY NOV 2003, vol. 12, no. 4, November 2003 (2003-11), pages 265-274, XP002553784 ISSN: 1055-8586
- GUTHMANN F. ET AL.: 'Plasma concentration of intestinal and liver-FABP in neonates suffering from necrotizing enterocolitis and in healthy preterm neonates' MOLECULAR AND CELLULAR BIOCHEMISTRY vol. 239, no. 1-2, 2002, pages 227 - 234, XP003018836
- FUJII H.: 'Lipid Biofactor no Kino Hatsugen ni Kan'yo suru Tanpakushitsugun no Kenkyu' KANAE IGAKU JOSEIKIN JUSHOSHA KENKYU GYOSEKISHU vol. 23RD, 01 October 1996, pages 419 - 431, XP003018837

## Description

### Technical Field

The present invention relates to a determination method and a determination reagent for acute enterocolitis.

### Background Art

Acute enterocolitis is one of the disease categories used for intestinal inflammation caused by external factors such as viruses, bacteria and pharmaceutical agents. The general symptoms thereof include abdominal pain and severe diarrhea, and the disease could be fatal depending on the cause.

For example, bacterial enterocolitis includes infectious diseases designated by law such as cholera and typhus abdominal, those originating from *Escherichia coli* 0157 which induces food-poisoning, and those caused by multiple drug resistant organisms such as MRSA. In addition, it is considered that viral enterocolitis is often caused by adenovirus, rotavirus, norovirus and the like. In other words, acute enterocolitis is not, in a precise sense, a diagnostic name of one disease, but a generic term for the syndromes that cause inflammations in the intestines. It is clinically clearly distinguished from endogenous enterocolitis, which is caused by genetic background, autoimmune diseases and the like.

Mild cases of acute enterocolitis are generally cured by medication and monitoring of the progress. However, once misdiagnosed, the symptoms progress and concurrently develop peritonitis due to gastrointestinal perforation and paralytic ileus, necessitating an operation or producing serious symptoms in some cases. Therefore, a quick and accurate diagnosis is demanded.

For the diagnosis of acute enterocolitis, (1) doctor's questions, (2) physical examination such as abdominal tenderness, diseased changes of skin, palpation and the like, (3) findings from laboratory examination such as blood test, scatoscopy and the like and (4) imaging examination such as abdomen plain X-ray, ultrasonication, CT, endoscopy, angiography and the like are used. What methods from among these are to be used and the combination thereof are mostly left to the judgment of the clinician based on the chief complaint and the clinical symptoms of the patient, and there is no definitive diagnostic procedure to arrive at a confirmed diagnosis. To be precise, acute enterocolitis is comprehensively judged not only from clinical symptoms such as diarrhea and abdominal pain, but from physical examination, laboratory examination, image examination and the like. As for the physical examination from those mentioned above, the inflammation site can be generally specified by palpation and abdominal tenderness after the onset of peritonitis. This requires proficient technique and experience. Stool culture, which is used as a laboratory examination to identify causative bacteria, is inappropriate for determining the treatment courses for patients in the acute stage since the test takes days. In addition, CT and endoscopy require facility having equipment and technicians, although the diagnostic accuracy is comparatively high, and endoscopy, which can most easily specify the inflammation site and level of severity, produces physical pain and burden of the patients. Therefore, endoscopy examination is not convenient diagnostic method.

In contrast, blood testing is frequently used as one of the diagnostic tools in clinical situations, since it minimizes the burden on the patients, provides various information of the patients quickly and conveniently, and has objectivity as the basis of diagnosis. For the diagnosis of acute enterocolitis, white blood cell count, C-reactive protein (CRP), lactic acid dehydrogenase (LDH) and the like are sometimes used as diagnostic markers. However, since they merely permit prediction of the presence of inflammation in the body with no organ specificity, they cannot be the basis of confirmed diagnoses of acute enterocolitis. In other words, a blood diagnosis marker capable of specifically and quickly identifying acute enterocolitis does not exist at present.

Therefore, there is a demand for establishment of a method of quickly and conveniently determining acute enterocolitis in clinical situations.

Non-patent reference 1, non-patent reference 2 and non-patent reference 3 describe that the concentration of intestinal fatty acid-binding protein (I-FABP) in the blood of patient with ischemic bowel disease increases than in healthy people. Furthermore, patent reference 1 and patent reference 2 describe that the I-FABP concentration of the blood of ischemic bowel disease rat model increases.

However, observed in those cases were only the pathologies showing markedly high I-FABP concentration as compared to that in a normal state, and therefore, the relationship between the changes in the blood I-FABP concentration and the severity of the disease was not established. In addition, they do not disclose that the blood I-FABP concentration increases due to the onset of acute enterocolitis.

Here, I-FABP is a specific protein locally distributed on the small intestinal mucosal epithelium, having a small molecular weight, and characteristically abundantly present in the cytoplasm fraction. In addition, it has functions to bind to fatty acid, and is involved in the intracellular metabolism of fatty acid.
patent reference 1: US Patent No. 5225329
patent reference 2: WO93/08276
non-patent reference 1: Tatsuo Kanda, Hiroshi Fujii, et al., Intestinal Fatty Acid-Binding Protein Is a Useful Diagnostic Marker for Mesenteric Infarction in Humans., "GASTROENTEROLOGY" 1996; 110: p. 339-343
non-patent reference 2: Joshua M. Liberman et al., Human intestinal fatty acid binding protein:Report of an assay with studies in normal volunteers and intestinal ischemia., "SURGERY" 1997; 121: No. 3: p. 335-342
non-patent reference 3: Florian Guthmann et al., Plasma concentration of intestinal-and liver-FABP in neonates suffering from necrotizing enterocolitis and in healthy preterm neonates., "Molecular and Cellular Biochemistry" 2002; 239: p. 227-234

### Disclosure of the Invention

### Problems to be Solved by the Invention

It is therefore an object of the present invention to provide a method of quickly, conveniently and accurately determining acute enterocolitis based on a quantitative and objective index, and a reagent for determining acute enterocolitis.

### Means of Solving the Problems

The present inventors have conducted intensive studies in an attempt to solve the above-mentioned problem, and developed a sandwich-type enzyme immunoassay using an anti-human I-FABP monoclonal antibody to enable high sensitive and precise detection of I-FABP in blood. The present inventors measured blood I-FABP concentration in acute enterocolitis rat experiment model in the I-FABP assay system, and found that the blood concentration significantly increased, based on which clarified that the blood I-FABP concentration of acute enterocolitis patients is high also in human clinical cases.

Furthermore, the present inventors have clarified that the blood I-FABP concentration of patients having abdominal pain or diarrhea not diagnosed as acute enterocolitis does not show difference from the blood I-FABP concentration of healthy subjects. Moreover, they have clarified that the blood I-FABP concentration shows a higher true positive rate than CRP and white blood cell count, which are existing diagnostic markers of inflammation in acute enterocolitis patients. Given these results and the organ specificity that I-FABP is present only on small intestinal mucosal epithelium, the present inventors have found that acute enterocolitis can be determined quickly, conveniently and accurately by measuring the blood I-FABP concentration, which resulted in the completion of the present invention.

Accordingly, the present invention provides the following.
[1] A method of determining acute enterocolitis, comprising detecting intestinal fatty acid-binding protein (I-FABP) in the blood collected from a mammal.
[2] The method of [1], which uses an anti-I-FABP monoclonal antibody.
[3] The method of [1] or [2], wherein the mammal is a human.
[4] The method of any one of [1] to [3], wherein the I-FABP in the blood is detected by an immunochemical method.
[5] The method of [4], wherein the immunochemical method is an enzyme immunochemical method, a latex agglutination method or an immunochromatography method.
[6] The method of claim [5], wherein the immunochemical method is an enzyme immunochemical method.
[7] The method of [6], wherein the enzyme immunochemical method is a sandwich-type enzyme immunoassay.
[8] The method of any one of [1] to [7], wherein the acute enterocolitis is infectious acute enterocolitis or acute enterocolitis due to an influence of a pharmaceutical agent.
[9] Use of an anti-I-FABP antibody for determining acute enterocolitis in a method in accordance with [1].
[10] Use according to [9], wherein a solid-phased anti-I-FABP antibody and an enzyme-labeled anti-I-FABP antibody are used in combination.
[11] Use according to [9] or [10], wherein the antibody is an anti-human I-FABP antibody.
[12] Use according to any of [9] to [11], wherein the antibody is a monoclonal antibody.
[13] Use according to any of [9] to [12], wherein the acute enterocolitis is infectious acute enterocolitis or acute enterocolitis due to an influence of a pharmaceutical agent.

### Effect of the Invention

According to the determination method and determination reagent of acute enterocolitis of the present invention, acute enterocolitis can be determined quickly and conveniently by detecting I-FABP in the blood, whereby objective and accurate determination results can be obtained. In addition, by monitoring the blood concentration over time, the pathological progress and treatment effects on acute enterocolitis can be determined.

### Brief Description of the Drawings

Fig. 1 is a graph showing the I-FABP concentration profile by the measurement of I-FABP in the blood of an acute enterocolitis rat experiment model of Example 2.
Fig. 2 shows a typical standard curve indicating the correlation between absorbance and human I-FABP concentration by an enzyme immunoassay using an anti-human I-FABP monoclonal antibody of Example 3.
Fig. 3 is a graph showing changes (average and dispersion) of I-FABP concentration of blood collected from 29 healthy subjects at 3 month intervals.

### Best Mode for Carrying out the Invention

The method of determining acute enterocolitis of the present invention is characterized by detection of I-FABP in blood. The I-FABP is released into the blood from the small intestinal mucosal epithelium of a mammal. In the present invention, blood to be used may be any of whole blood, serum and plasma, which can be obtained as appropriate by treating, according to a conventional method, the blood taken from a mammal. Here, human acute enterocolitis can be determined when the blood is human blood, and the I-FABP is human I-FABP. In the present specification, "I-FABP detection" includes all of quantitative measurement of I-FABP concentration, semiquantitative measurement of I-FABP concentration within a given range, and qualitative detection of the presence or absence of I-FABP at a given concentration or above.

The detection method of I-FABP is not limited to a particular method and, for example, immunochemical methods, various chromatography methods such as HPLC method and the like can be employed. Particularly, since acute enterocolitis can be accurately determined by detecting I-FABP in the blood by an immunochemical method, an immunochemical method is preferable. The immunochemical method is not limited to a particular method and, for example, enzyme immunochemical method, latex agglutination method, immunochromatography method, radioimmunoassay, fluorescence immunoassay, luminescent immunoassay, spin immunoassay, turbidimetry, enzyme sensor electrode method, immunoelectrophoresis, Westernblot method and the like can be used. Of these, enzyme immunochemical method, latex agglutination method and immunochromatography method are preferable since they achieve high sensitivity. Furthermore, the enzyme immunochemical method may be a competitive method. However, a sandwich-type enzyme immunoassay, which is one kind of non-competitive method, is more preferable since the I-FABP concentration in blood can be measured with high sensitivity and high precision by an easy operation.

In the sandwich-type enzyme immunoassay, I-FABP is sandwiched between two kinds of antibodies that recognize different epitopes present in I-FABP, namely, I-FABP is sandwiched between a solid-phased anti-I-FABP antibody and an enzyme-labeled anti-I-FABP antibody. Then the enzyme amount of the labeled antibody bound with I-FABP is measured to determine the I-FABP concentration.

In addition, as one kind of the sandwich-type enzyme immunoassay, a method utilizing an avidin-biotin reaction can also be used. According to this method, I-FABP concentration is measured by performing an antigen antibody reaction between the I-FABP trapped by a solid-phased anti-I-FABP antibody and biotin-labeled anti-I-FABP antibody, and then adding enzyme-labeled streptavidin thereto, and measuring the enzyme amount of the labeled antibody bound to I-FABP.

The latex agglutination method utilizes an agglutination reaction of antibody sensitized latex particles and antigen. According to this method, I-FABP concentration is determined by measuring the agglutination level of the latex particles produced by an immunoreaction between anti-I-FABP antibody sensitized latex particles and I-FABP.

In the immunochromatography method, a sheet-like carrier carrying all immunochemical reactions. Since this method does not require a special measurement device, it is an advantageous method when a quick determination is needed for judgment outside the hospital, in an emergency situation and the like. In addition, this method is suitable for qualitatively detecting the presence or absence of I-FABP at a concentration of not less than an arbitrarily-set level, namely, a cutoff value.

In the immunochromatography method, when the blood as a sample is added dropwise to a carrier, I-FABP in the blood and an anti-I-FABP antibody labeled with colloidal gold and the like performs an immunoreaction to form an immunocomplex. The complex is developed on the carrier, trapped by an anti-I-FABP antibody recognizing a different epitope, which is solid-phased on a particular region of the carrier, and the labeling substances accumulate. By visually observing the accumulation level, the I-FABP concentration is measured.

The reagent for determining acute enterocolitis of the present invention comprises an anti-I-FABP antibody that recognizes and specifically reacts with I-FABP, so that the method of determining acute enterocolitis of the present invention can be practiced by an immunochemical method. While the anti-I-FABP antibody may be a polyclonal antibody, a monoclonal antibody is more preferably used since it has high specificity and can stably supply homogeneous products. When the target is human, an anti-human I-FABP antibody is used. When a monoclonal antibody is to be used, it is preferable to confirm in advance that the epitopes of solid-phased anti-I-FABP antibody and labeled anti-I-FABP antibody are different.

The antibody to be used in the present invention can be produced by a conventional method. Polyclonal antibody can be obtained by immunizing animals such as rabbit, mouse, goat, horse and the like with I-FABP. In addition, monoclonal antibody can be obtained by fusing the spleen cell of an animal immunized with I-FABP and myeloma, cloning the cells to select a hybridoma, and culturing the hybridoma. The I-FABP as an antigen to be used for producing an anti-I-FABP antibody is obtained by, for example, purification from an extract of a small intestinal mucosal epithelium, as described in Tatsuo Kanda, Teruo Ono et al., Possible role of rat fatty acid-binding proteins in the intestine as carriers of phenol and phthalate derivatives., "BIOCHEMICAL AND BIOPHYSICAL RESEARCH COMMUNICATIONS" 1990; 168: p.1053-1058. However, it can also be obtained by cell culture or gene recombination technology. In addition, a region peptide of I-FABP can also be used as an antigen.

When the determination reagent of the present invention is based on a sandwich-type enzyme immunoassay, the anti-I-FABP antibody thus obtained is contained in the reagent in the form of an enzyme-labeled anti-I-FABP antibody and a solid-phased anti-I-FABP antibody.

Here, while the labeling substance to be bound to an anti-I-FABP antibody to give an enzyme-labeled anti-I-FABP antibody is not limited to a particular one, enzymes such as peroxidase, β-galactosidase, alkaline phosphatase and the like can be used. A labeling substance can be bound to an antibody according to a conventional method and using a carboxyl group, an amino group, a thiol group, a hydroxyl group and the like that they have.

A solid-phased anti-I-FABP antibody can be produced by binding an anti-I-FABP antibody to a solid phase such as microplate well, plastic beads and the like. The binding to a solid phase generally involves dissolving an antibody in a suitable buffer such as citrate buffer and the like to establish a contact between the surface of the solid phase and the antibody solution for a suitable time (1 - 2 days). Furthermore, to suppress non-specific adsorption and non-specific reaction, a phosphate buffer solution, in which bovine serum albumin (BSA), bovine milk protein and the like are dissolved, is contacted with a solid phase to block, with the aforementioned BSA, bovine milk protein and the like, the surface of the solid phase where the antibody failed to coat.

The enzyme immunoassay and enzyme immunoassay reagent used for detecting I-FABP are described in, for example, Tatsuo Kanda, Hiroshi Fujii, et al., Intestinal Fatty Acid-Binding Protein Is a Useful Diagnostic Marker for Mesenteric Infarction in Humans., "GASTROENTEROLOGY" 1996; 110: p. 339-343. In addition, reagents for an immunochromatography method and latex agglutination method are general in this field, and can be produced by applying the anti-I-FABP antibody obtained as mentioned above to these reagents.

Furthermore, by measuring the blood I-FABP concentration by, for example, an enzyme immunochemical method using an anti-I-FABP antibody, acute enterocolitis can be objectively and accurately diagnosed, and quantitative evaluation of the severity of acute enterocolitis and the evaluation of the pathological progression over time become possible. To be specific, acute enterocolitis is diagnosed when the blood I-FABP concentration is higher than a given level. The severity of acute enterocolitis is determined according to the given level, and the pathological progress over time is evaluated based on the time course of the I-FABP concentration.

Acute enterocolitis is a generic term for syndromes causing intestinal inflammation with diarrhea and abdominal pain, and includes enterocolitis due to infection with bacterium, virus and the like, enterocolitis due to physicochemical stimulation such as excessive consumption of alcohol, drug stimulation and the like. The determination method and determination reagent for acute enterocolitis of the present invention are widely applicable to the determination of acute enterocolitis including these syndromes.

The determination reagent of the present invention is provided to the market as a commercial package. The commercial package of the present invention contains the above-mentioned determination reagent of acute enterocolitis and a written matter relating to the reagent, wherein the written matter and/or the package state that the reagent can or should be used for the determination of acute enterocolitis. With this constitution, the use of the determination reagent of the present invention can be certainly known to the user and the like. The package may additionally contain, in a kit, a reagent used for determination of acute enterocolitis, for example, a buffer usable for dilution, washing and the like, a coloring reagent (substrate fluid) and the like when the determination is based on a sandwich-type enzyme immunoassay.

While the present invention is explained in detail in the following by referring to specific examples, the technical scope of the present invention is not limited by the examples. Examples

### Example 1

### (A) [Acquirement of rabbit anti-rat I-FABP polyclonal antibody]

An anti-rat I-FABP polyclonal antibody was acquired by the following procedure.

Small intestinal mucosa was collected from 60 SD rats (male) and, after homogenate extraction with Tris-HCl buffer (0.1 mol/L, pH 7.4, containing 1 mmol/L EDTA), purified by Sephadex G-75, DEAE-cellulose and Hydroxyapatite column to give purified rat I-FABP (15 mg). This was used as an antigen to immunize a rabbit. The amount of the antigen used was 100 µg for the first time and 50 µg for the second time onwards, which was suspended in Freund's adjuvant and used. The antibody titer of the rabbit serum was confirmed by gel double immunity diffusion method, and whole blood was collected and centrifuged at 3000 rpm × 10 min to prepare the anti-serum.

From the obtained anti-serum, an anti-rat I-FABP specific polyclonal antibody could be obtained by Sepharose 6MB chromatography using purified rat I-FABP solid-phased as a ligand. The obtained polyclonal antibody was examined for the reactivity with the cytoplasmic fractions of the liver, jejunum, heart and stomach prepared from a Wistar rat (male) by analysis by the Westernblot method. As a result, it showed strong reactivity with a position corresponding to 15 kDa derived from the jejunum, showed slight reactivity with the same fraction derived from the stomach, and showed no reactivity with the fractions derived from the liver and the heart. Therefore, it was shown that the present antibody is specific to rat I-FABP, and does not show cross-reactivity with Heart type (H-) FABP and hepatic (L-) FABP, which are structurally similar.

### (B) [Enzyme immunoassay using anti-rat I-FABP polyclonal antibody]

Using the anti-rat I-FABP polyclonal antibody acquired in the aforementioned (A), the blood I-FABP concentration of a rat was measured by a sandwich-type enzyme immunoassay.

The standard curve of the rat I-FABP measurement was drawn by the following procedure.

Purified rat I-FABP was appropriately diluted to give rat I-FABP solutions of various concentrations. Each of the diluted standard solutions was added by 100 µL to a solid-phased anti-rat I-FABP antibody coated wells, stirred in a mixer, and incubated at 37°C for a given time. Then, the reaction mixture was removed from each well using an ELISA washer, and each well was washed with a washing solution (0.3 mL) prepared by diluting the undiluted washing solution. The washing operation was repeated 3 times, and the washing solution remaining in the well was removed with a paper towel and the like.

A solution (100 µL) of an anti-rat I-FABP polyclonal antibody labeled with horseradish peroxidase was added to a reaction well, incubated at room temperature for a given time, and a washing operation was performed in the same manner as above. A substrate solution (100 µL) containing o-phenylenediamine (33 mg/mL) was added, and the mixture was incubated in a dark place at room temperature for precisely 15 min. Then, a reaction stopping solution (100 µL) consisting of 2N sulfuric acid was added and the mixture was rapidly stirred in a mixer to quench the enzyme reaction. The absorbance at 492 nm was measured for each well and a standard curve was drawn.

It was reproducibly shown that the absorbance obtained by the above-mentioned operation is in a linear correlation with rat I-FABP value. By measuring the absorbance in the same manner as above, the rat I-FABP value in blood samples can be accurately determined from the standard curve.

### Example 2

### [Measurement of blood I-FABP in acute enterocolitis rat experimental model]

The abdomen of SD rats (male) was opened under anesthesia and a jejunum ligation loop (15 cm) was prepared. They were divided into 3 groups: control group (n=8), *V. cholerae* group (n=8) and *Cl. difficile* group (n=8). Cholerae toxin (30 µg) for the *V. cholerae* group and Difficile A toxin 5×2¹⁹ CU (Cytotoxic Unit) for the *Cl. difficile* group were dissolved in saline and injected into the respective loops. Blood samples were collected at 2, 4, 6 and 8 hours after the close of abdominal region, and the I-FABP level in serum was measured by a sandwich-type enzyme immunoassay using the anti-rat I-FABP polyclonal antibody described in Example 1.

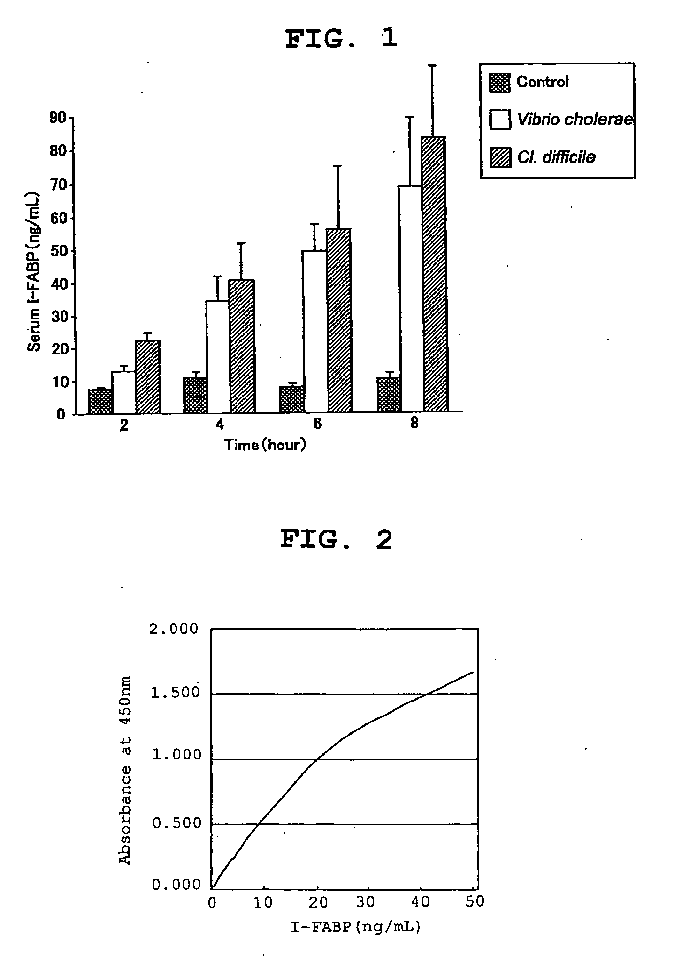

The measurement results are shown in Fig. 1. The I-FABP level in serum was about 10 ng/mL in the control group throughout the entire progress. However, the I-FABP level increased with time in the enterocolitis group, and the 8 hr level was 68.2 ng/mL for the *V. cholerae* group and 82.4 ng/mL for the *Cl. difficile* group, both showing a significant increase.

From the foregoing, since I-FABP increases in acute enterocolitis rat experimental models, it was confirmed that acute enterocolitis can be objectively determined by measuring the blood I-FABP concentration.

### Example 3

### (A-1) [Acquirement of rabbit anti-human I-FABP polyclonal antibody]

NZW rabbit (male) was immunized, at 2-week intervals, with 100 µg/rabbit for the first time and 50 µg/rabbit for the second time onwards of recombinant I-FABP (rI-FABP) obtained by expressing human I-FABP gene by baculovirus. After immunization, an aliquot of blood was sampled, and the degree of antibody titer was confirmed with the reactivity with solid-phased rI-FABP as an index. Then, whole blood was collected from the carotid artery under anesthesia, and the obtained blood was centrifuged at 3000 rpm × 10 min and the blood cell fraction was removed to give anti-serum. The obtained anti-serum was subjected to ammonium sulfate fractionation, DEAE-cellulose purification and affinity purification to give a rabbit anti-human I-FABP polyclonal antibody.

### (A-2) [Acquirement of mouse anti-human I-FABP monoclonal antibody]

rI-FABP obtained by expressing human I-FABP gene by baculovirus was dissolved in saline, suspended in Freund's adjuvant and used. BALB/c mouse (female) was immunized, at 2-week intervals, with 100 µg/mouse for the first time and 50 µg/mouse for the second time onwards thereof. After immunization, an aliquot of blood was sampled, and the degree of increase in the antibody titer at an anti-serum level was confirmed with the reactivity with solid-phased rI-FABP as an index.

Then, spleen cells of a mouse with a sufficiently increased antibody titer were collected. Mouse myeloma cultured in advance and the spleen cells were mixed at a ratio of 1:2 - 1:10, and the cells were fused by the PEG method. The fused cells were seeded on a culture plate by a limit dilution method. Thereafter, the cells were cultured in a CO₂ gas incubator at 37°C for 7 - 20 days.

Next, hybridoma culture supernatant was collected, and the titer was confirmed with the reactivity with a solid-phased rI-FABP as an index. Positive clone cells were passage cultured, and cloned by a limiting dilution method. The obtained cells derived from a single colony were used as anti-human I-FABP monoclonal antibody producing hybridoma.

Then, the obtained monoclonal antibody producing hybridoma was cultured in large scale. Pristane (1 mL) was intraperitoneally administered in advance, and about 5×10⁶ - 2×10⁷ cells of hybridoma were administered to a BALB/c mouse (female) preliminarily raised for not less than 2 weeks. The mouse was raised for 10 - 25 days while awaiting accumulation of the ascitic fluid. Thereafter, the ascitic fluid was collected, and the obtained ascitic fluid antibody was subjected to ammonium sulfate fractionation, DEAE-cellulose purification and affinity purification to give a mouse anti-human I-FABP monoclonal antibody.

### (B) [Enzyme immunoassay using anti-human I-FABP monoclonal antibody]

Using the kit constituted with the following reagents, an enzyme immunoassay by a sandwich-type enzyme immunoassay was performed. While the monoclonal antibody obtained in the aforementioned (A-2) is used here, the polyclonal antibody obtained in (A-1) can also be used instead. The contents of each of the following reagents are generally used in the present field.

(1) Standard reagent: A protein solution containing I-FABP prepared according to the aforementioned (A-1) or (A-2), and those prepared to 1, 2.5, 5, 10, 25, 50 (ng/mL) are used. A solution having the identical composition and free of human I-FABP is used as standard reagent 0.
(2) Sample dilution solution: A protein solution having a buffering ability at near neutral. This solution is used as it is.
(3) Solid-phased anti-I-FABP antibody coated well: A 96 well microplate well made of polystyrene, which is coated with anti-human I-FABP monoclonal antibody. The well is used as it is. One well is used for one measurement.
(4) Enzyme-labeled antibody reagent: An enzyme-labeled anti-human I-FABP monoclonal antibody labeled with horseradish peroxidase (HRP), which is diluted to a concentration for use with a protein solution having a buffering ability at near neutral and added with a preservative and the like. This reagent is used as it is. 10 µL of the stock solution before dilution is used for one measurement.
(5) Washing solution: A solution containing buffer components. A detergent is added as appropriate to enhance the washing effect.
(6) Substrate reagent: A solution containing tetramethylbenzidine (TMB). The substrate reagent is used as it is. 100 µL of the substrate reagent is used for one measurement.
(7) Reaction stopping solution: A solution containing sulfuric acid. The solution is used as it is. 100 µL of the reaction stopping solution is used for one measurement.

First, a standard curve was drawn according to the following procedure. A standard reagent was appropriately diluted with a sample dilution solution to give human I-FABP solutions of various concentrations. Each of the diluted standard solutions was added by 100 µL to a solid-phased anti-human I-FABP antibody binding well, stirred in a mixer, and incubated at room temperature for a given time. Then, the reaction mixture was removed from each well using an ELISA washer, and each well was washed with a washing solution (0.3 mL) prepared by diluting the undiluted washing solution. The washing operation was repeated 3 times, and the washing solution remaining in the well was removed with a paper towel and the like.

An enzyme labeled antibody reagent (100 µL) was added to a reaction well, incubated at room temperature for a given time, and a washing operation was performed in the same manner as above. A substrate reagent (100 µL) was added, and the mixture was incubated in a dark place at room temperature for precisely 30 min. Then, a reaction stopping solution (100 µL) was added and the mixture was rapidly stirred in a mixer to quench the enzyme reaction. The absorbance at 450 nm was measured for each well and a standard curve was drawn.

An example of a typical standard curve showing the correlation between the absorbance obtained by the above-mentioned operation and human I-FABP value is shown in Fig. 2. By measuring the absorbance in the same manner as above and using a sample instead of the diluted standard reagent, human I-FABP value in the sample can be accurately determined within the range of 0.1 - 50 ng/mL from the standard curve.

In fact, when two I-FABP added samples were sequentially diluted and the dilution rate and the measured values were plotted, a straight line almost passing through the point of origin was obtained, and the linearity of dilution was good.

In addition, two I-FABP added samples and the standard solution were admixed at a given ratio, and the recovery rate relative to the addition concentration was calculated. As a result, the rate was almost 90 - 110% and the addition recovery rate was good.

Furthermore, three I-FABP added samples were subjected to an intra-assay reproducibility test by repeating 8 times. As a result, the coefficient of variation (CV%) was 0.9 - 3.9%. In addition, the same samples were subjected to an inter-assay reproducibility by repeating 4 times. As a result, the coefficient of variation (CV%) was 3.1 - 4.9%, and the reproducibility of tests was good.

As mentioned above, the test results of dilution linearity, addition recovery rate and reproducibility were all good, and the enzyme immunoassay using the anti-human I-FABP monoclonal antibody in the present example was confirmed to be highly reliable.

### Example 4

### [Measurement of blood I-FABP in clinical cases]

About 1 mL of blood was drawn from patients and serum was separated. Using the obtained serum as a sample, an enzyme immunoassay was performed using the anti-human I-FABP monoclonal antibody described in Example 3. As shown in each of the following clinical cases, the I-FABP concentration was high in acute enterocolitis patients.

In clinical cases, therefore, acute enterocolitis can be determined objectively, accurately, quickly and conveniently by measuring the blood I-FABP concentration. In addition, determination of the pathological progression and treatment effect of acute enterocolitis becomes possible by monitoring the blood concentration over time.

The detection limit of the I-FABP concentration of human serum by the present evaluation system is 0.1 ng/mL, and the I-FABP concentration of healthy subjects in Example 5 was around 1 ng/mL.

In contrast, in the animal experiment shown in Example 2, the concentration of the control group became as high as about 10 ng/mL. The reasons for this difference are considered to be that the detection sensitivity is different due to the difference between polyclonal antibody and monoclonal antibody, the normal range may be different between human and rat, an influence of laparotomy performed for preparing a closed small intestinal loop in a rat experiment is present, and the like.

Moreover, the blood concentration of 20 - 65 ng/mL of a healthy subjects has been reported in Tatsuo Kanda, Hiroshi Fujii, et al., Intestinal Fatty Acid-Binding Protein Is a Useful Diagnostic Marker for Mesenteric Infarction in Humans., "GASTROENTEROLOGY" 1996; 110: p. 339-343. The difference from the results of the present experiment is considered to be attributable to the different detection sensitivity due to the difference in antibodies, and the like.

### (1) Clinical case 1

A 78-year-old female. Abdominal pain emerged from night, gradually became stronger, and started to accompany diarrhea, vomiting and fever. The next day, the patient was taken to the hospital by ambulance and hospitalized. By the test run at the time of admission, increased leukocytes (11,700/mm³) and mild metabolic acidosis (BE, -1.9) were found. Clinical diagnosis was moderately severe acute enterocolitis, and fluid replacement by drip infusion was performed under fasting. Later, the symptoms were resolved gradually, and the patient was discharged from the hospital 2 days after the admission. When an enzyme immunoassay using an anti-human I-FABP monoclonal antibody was performed, the I-FABP value at the time of admission was 10 ng/mL, and that on the day of discharge was 0.8 ng/mL. Since the I-FABP values of healthy subjects are around 1 ng/mL, increased I-FABP value of serum when symptoms were present and value change corresponding to the symptom profile were observed.

### (2) Clinical case 2

A 56-year-old female. Abdominal pain and diarrhea emerged. Since the symptoms did not improve, she visited a clinic as an outpatient 2 days after the symptoms emerged. No abnormality was found except a high CRP value (4.67) by the blood test at the time of visit as an outpatient. Clinically diagnosis was mild acute enterocolitis, and intestinal remedy and anticonvulsant were prescribed. The symptoms disappeared in several days.

When an enzyme immunoassay using an anti-human I-FABP monoclonal antibody was performed, the I-FABP value at the time of visit as an outpatient was 2.2 ng/mL, which was slightly higher than that of a healthy person (around 1 ng/mL). The milder symptom as compared to clinical case 1 is correlated with the comparatively small degree of increase in the I-FABP concentration. According to the method of the present invention, therefore, pathological progress and changes in the treatment effect over time of acute enterocolitis can be known accurately.

### Example 5

### [Relation between I-FABP distribution and abdominal pain diarrhea in healthy subjects]

59 serum samples from healthy volunteers were measured in an attempt to find the I-FABP concentration range of sera in healthy subjects. For selection of healthy subjects, 6 items were set as criteria (1. nondiabetic, 2. normal renal function, 3. non-hyperlipidemic, 4. no history of small intestine resection, 5. no active small intestinal disease, and 6. normal hepatic function). In addition, a search was conducted on the day of blood drawing as to the onset of abdominal pain and diarrhea in the past 3 days, and the influence of these symptoms on the blood I-FABP concentration was examined. The 59 healthy volunteers consisted of 41 males (25 - 50 years old, 37.3±6.2 years old) and 18 females (28 - 58 years old, 39.8±8.1 years old). Among them, the test subjects who experienced symptoms of abdominal pain or diarrhea in the past 3 days were 2 for abdominal pain alone; 3 for diarrhea alone; and 2 for both abdominal pain and diarrhea.

Blood samples (about 5 mL) were collected from healthy volunteers, and serum fractions were obtained by centrifugation. The I-FABP concentration in the obtained sera was quantified in the same manner as in Example 4 by an enzyme immunoassay using an anti-human I-FABP monoclonal antibody.

The I-FABP measurement values and distribution thereof in all cases including abdominal pain and diarrhea are shown in Table 1. As shown in the Table, mean±S.D. of blood I-FABP concentration of 59 samples was 1.02±0.53 ng/mL (male: 1.03±0.53 ng/mL, female: 0.97±0.52 ng/mL). A statistical study of the influence of sex and age revealed no factor that had influenced blood I-FABP concentration (Table 2).

**[Table 1]**

| Information of healthy subjects and statistics of blood I-FABP concentration | | | | | |
|---|---|---|---|---|---|
| | | Age | | I-FABP | |
| group | n | average | S.D. | average | S.D. |
| total | 59 | 38.1 | 6.8 | 1.02 | 0.53 |
| male | 41 | 37.3 | 6.2 | 1.03 | 0.53 |
| female | 18 | 39.8 | 8.1 | 0.97 | 0.52 |

**Table 2**

| Statistics relating to variation of blood I-FABP concentration by sex and age | | | | | |
|---|---|---|---|---|---|
| | | sex | | age | |
| | n | p value | | r | |
| total | 59 | 0.688 | n.s | 0.151 | n.s |

Next, an influence of symptoms of abdominal pain or diarrhea on the blood I-FABP concentration was studied. As shown in Table 3, neither showed a statistically significant difference, and the symptoms of abdominal pain and diarrhea did not influence the blood I-FABP concentration. In other words, it was clarified that the blood concentration does not change in mild symptoms that do not require clinical diagnosis, such as abdominal pain and like.

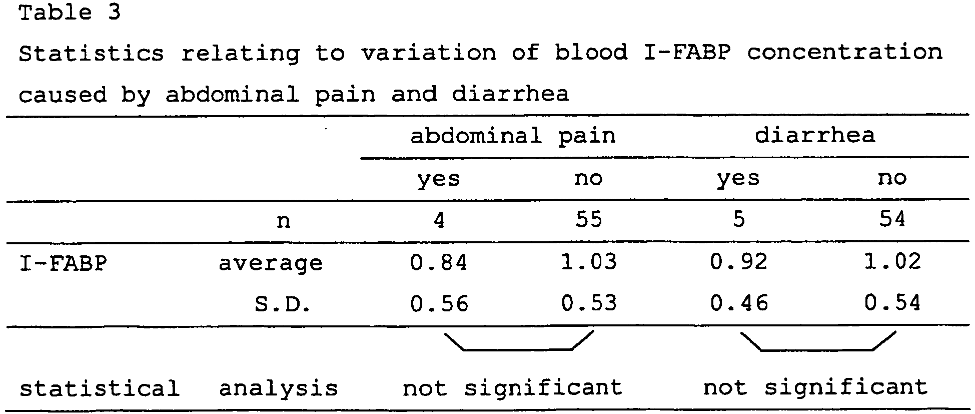

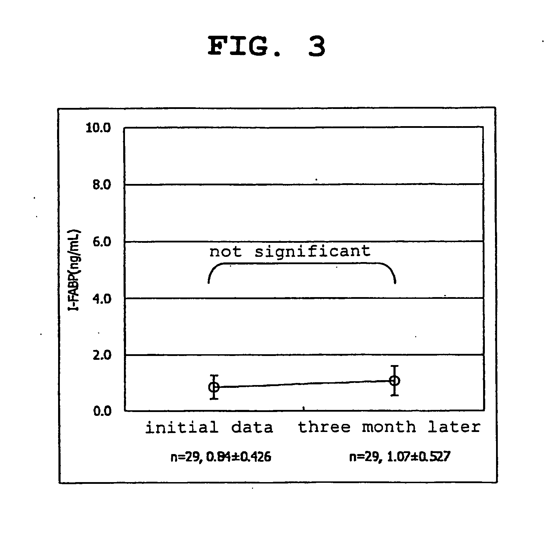

On the other hand, 29 volunteers were randomly selected from the aforementioned 59 volunteers, and the blood I-FABP concentration was measured again 3 months later. The variation in the measured values of the test subjects at that time is shown in Fig. 3. As shown in Fig. 3, although the blood concentration of the same test subject was not constant all the time, the range of variation thereof was small, and did not exceed mean±2 S.D. of the blood concentration of healthy subjects in a 95% confidence interval.

From the foregoing, it was clarified that the blood I-FABP concentration of a healthy person is mostly constant without an influence of sex difference and aging, and is around 1 ng/mL. In addition, this value did not change significantly in abdominal pain and diarrhea of the level that is not diagnosed as acute enterocolitis. Hence, it was clarified that acute enterocolitis can be diagnosed extremely accurately by measuring the blood I-FABP concentration.

### Example 6

### [Comparison of true positive rate in acute enterocolitis patients]

In 10 cases clinically diagnosed as acute enterocolitis, serum I-FABP concentration was measured using an anti-human I-FABP monoclonal antibody. C reactive protein (CRP) and white blood cell count (WBC), which are general inflammation markers, were also measured for the same samples, and the efficiency in acute enterocolitis diagnosis was compared.

As a result, as shown in Table 4, while the true positive rate of conventional inflammation markers, CRP and WBC, was 70% for CRP and 50% for WBC, I-FABP showed an extremely high diagnostic sensitivity of 90%. The standard value (less than 2.0 ng/mL) used in this experiment for the diagnosis of I-FABP was calculated from the confidence interval 95% of the blood I-FABP concentration of a healthy subject shown in Example 5.

Accordingly, it was clarified that the diagnosis of acute enterocolitis by measuring the blood I-FABP concentration has not only organ specificity to the small intestine but also high usefulness as compared to CRP and white blood cell count, which are general diagnosis markers for inflammation.

**[Table 4]**

| Comparison of I-FABP concentration and inflammation markers in determination of disease in acute enterocolitis cases | | | | | | |
|---|---|---|---|---|---|---|
| | I-FABP (<2.0) | | CRP (<0.3) | | WBC (3500-9000) | |
| Case No. | (ng/ml) | determination | (mg/dL) | determination | (/mm³) | determination |
| 1 | 12.4 | + | 1.63 | + | 9800 | + |
| 2 | 11.0 | + | 0.46 | + | 11500 | + |
| 3 | 10.0 | + | 0.44 | + | 12700 | + |
| 4 | 3.8 | + | 0.07 | - | 7600 | - |
| 5 | 3.1 | + | 0.08 | - | 3000 | - |
| 6 | 2.7 | + | 2.37 | + | 12600 | + |
| 7 | 2.5 | + | 7.16 | + | 8700 | - |
| 8 | 2.5 | + | 5.83 | + | 8700 | - |
| 9 | 2.1 | + | 4.19 | + | 3700 | - |
| 10 | 1.8 | - | 0.01 | - | 15700 | + |
| true positive rate | 90% | | 70% | | 50% | |

## Claims

1. A method of determining acute enterocolitis, comprising detecting intestinal fatty acid-binding protein (I-FABP) in the blood collected from a mammal.

2. The method of claim 1, which uses an anti-I-FABP monoclonal antibody.

3. The method of claim 1 or 2, wherein the mammal is a human.

4. The method of any one of claims 1 to 3, wherein the I-FABP in the blood is detected by an immunochemical method.

5. The method of claim 4, wherein the immunochemical method is an enzyme immunochemical method, a latex agglutination method or an immunochromatography method.

6. The method of claim 5, wherein the immunochemical method is an enzyme immunochemical method.

7. The method of claim 6, wherein the enzyme immunochemical method is a sandwich-type enzyme immunoassay.

8. The method of any one of claims 1 to 7, wherein the acute enterocolitis is infectious acute enterocolitis or acute enterocolitis due to an influence of a pharmaceutical agent.

9. Use of an anti-I-FABP antibody for determining acute enterocolitis in a method in accordance with claim 1.

10. Use according to claim 9, wherein a solid-phased anti-I-FABP antibody and an enzyme-labeled anti-I-FABP antibody are used in combination.

11. Use according to claim 9 or 10, wherein the antibody is an anti-human I-FABP antibody.

12. Use according to any of claims 9 to 11, wherein the antibody is a monoclonal antibody.

13. Use according to any of claims 9 to 12, wherein the acute enterocolitis is infectious acute enterocolitis or acute enterocolitis due to an influence of a pharmaceutical agent.

## Patentansprüche

1. Verfahren zum Feststellen akuter Enterocolitis, umfassend das Nachweisen von intestinalem Fettsäure-bindenden Protein (I-FABP) in Blut, das von einem Säuger erhalten wurde.

2. Verfahren nach Anspruch 1, das einen monoclonalen Anti-I-FABP-Antikörper verwendet.

3. Verfahren nach Anspruch 1 oder 2, wobei der Säuger ein Mensch ist.

4. Verfahren nach einem der Ansprüche 1 bis 3, wobei das I-FABP im Blut durch ein immunchemisches Verfahren nachgewiesen wird.

5. Verfahren nach Anspruch 4, wobei das immunchemische Verfahren ein enzymimmunchemisches Verfahren, ein Latexagglutinationsverfahren oder ein Immunchromatographieverfahren ist.

6. Verfahren nach Anspruch 5, wobei das immunchemische Verfahren ein enzymimmunchemisches Verfahren ist.

7. Verfahren nach Anspruch 6, wobei das enzymimmunchemische Verfahren ein Enzymimmuntest vom Sandwich-Typ ist.

8. Verfahren nach einem der Ansprüche 1 bis 7, wobei die akute Enterocolitis eine infektiöse akute Enterocolitis oder eine durch den Einfluss eines pharmazeutischen Wirkstoffs bedingte akute Enterocolitis ist.

9. Verwendung eines Anti-I-FABP-Antikörpers zum Nachweisen einer akuten Enterocolitis in einem Verfahren gemäß Anspruch 1.

10. Verwendung nach Anspruch 9, wobei ein Festphasen-Anti-I-FABP-Antikörper und ein enzymmarkierter Anti-I-FABP-Antikörper in Kombination verwendet werden.

11. Verwendung nach Anspruch 9 oder 10, wobei der Antikörper ein Anti-human-I-FABP-Antikörper ist.

12. Verwendung nach einem der Ansprüche 9 bis 11, wobei der Antikörper ein monoclonaler Antikörper ist.

13. Verwendung nach einem der Ansprüche 9 bis 12, wobei die akute Enterocolitis eine infektiöse akute Enterocolitis oder eine durch den Einfluss eines pharmazeutischen Wirkstoffs bedingte akute Enterocolitis ist.

## Revendications

1. Procédé de détermination d'une entérocolite aiguë, comprenant la détection de la protéine de liaison aux acides gras des intestins (I-FABP) dans le sang prélevé d'un mammifère.

2. Procédé selon la revendication 1, qui utilise un anticorps monoclonal anti-I-FABP.

3. Procédé selon la revendication 1 ou 2, dans lequel le mammifère est un être humain.

4. Procédé selon l'une quelconque des revendications 1 à 3, dans lequel l'I-FABP dans le sang est détectée par un procédé immunochimique.

5. Procédé selon la revendication 4, où le procédé immunochimique est un procédé immunochimique enzymatique, un procédé d'agglutination au latex ou un procédé d'immunochromatographie.

6. Procédé selon la revendication 5, où le procédé immunochimique est un procédé immunochimique enzymatique.

7. Procédé selon la revendication 6, où le procédé immunochimique enzymatique est un test immune-enzymatique de type sandwich.

8. Procédé selon l'une quelconque des revendications 1 à 7, dans lequel l'entérocolite aiguë est une entérocolite aiguë infectieuse ou une entérocolite aiguë due à l'influence d'un agent pharmaceutique.

9. Utilisation d'un anticorps anti-I-FABP pour la détermination d'une entérocolite aiguë dans un procédé conformément à la revendication 1.

10. Utilisation selon la revendication 9, où un anticorps anti-I-FABP en phase solide et un anticorps anti-I-FABP marqué par une enzyme sont utilisés en combinaison.

11. Utilisation selon les revendications 9 ou 10, où l'anticorps est un anticorps anti-I-FABP humaine.

12. Utilisation selon l'une quelconque des revendications 9 à 11, où l'anticorps est un anticorps monoclonal.

13. Utilisation selon l'une quelconque des revendications 9 à 12, où l'entérocolite aiguë est une entérocolite aiguë infectieuse ou une entérocolite aiguë due à l'influence d'un agent pharmaceutique.
